# EUROPEAN PATENT SPECIFICATION

(11) **EP 3 515 376 B1**
(45) Date of publication and mention of the grant of the patent: **12.02.2025**
(21) Application number: 17785039.3
(22) Date of filing: 21.09.2017
(51) Int. Cl.: A61F 5/01

(54) **ANKLE SUPPORT**
SPRUNGGELENKSTÜTZE
SUPPORT DE CHEVILLE

(30) Priority: 22.09.2016 NL 2017512
(43) Date of publication of application: 31.07.2019
(73) Proprietor: Nea International B.V., 6199 AB Maastricht-Airport (NL)
(72) Inventor: VOSKUILEN, Agnes Theodora Maria, 6199 AB Maastricht-Airport (NL); KOESLAG, Jeroen Hendrik Johan, 6199 AB Maastricht-Airport (NL)
(74) Representative: Algemeen Octrooi- en Merkenbureau B.V.
(86) International application number: PCT/NL2017/050625
(87) International publication number: WO 2018/056812

(56) References cited:
- EP-A1- 0 893 112
- EP-A1- 1 138 288
- DE-A1- 10 019 341
- DE-A1- 19 854 536
- FR-A1- 2 993 452
- US-A- 4 715 131
- US-A- 4 729 370
- US-A- 5 899 872

## Description

The invention relates to an ankle support having a lateral side and a medial side, wherein the ankle support at least comprises a one-piece support frame and at least two draw straps which, when the ankle support is arranged on the foot, each run along different sides of the lower leg, wherein the one-piece support frame comprises a top section and a connection section, the connection section connects the top section with the bottom section on the medial side of the ankle support, such that, when the ankle support is arranged on the lower leg, the ankle and the foot, the top section of the support frame is located above the medial malleolus. Further, the invention relates to a method for manufacturing an ankle support.

FR-2.993.452 discloses a one-piece ankle stabilizing orthosis comprising two uprights and a sole. Two bands connect the two uprights to the sole.

EP-1.138.288 discloses such an ankle support. This ankle support is able to prevent inversion of the foot. The ankle support ensures that in the event of inversion of the foot the draw straps, in conjunction with the support element, prevent further movement in the direction associated with the inversion. The movement associated with the inversion is translated into the application of a tensile load to the draw straps, which directly transmit this load to a top section of the support element preventing further movement of the foot in the inversion direction. The known structure is lightweight, not very bulky and does not limit or scarcely limits the freedom of movement in directions other than that of an inversion.

It is an object of the present invention to improve the known ankle support. This object is achieved by an ankle support having the features of claim 1.

The bottom section of the support frame comprises a bottom medial section connected to the connection section, a bottom lateral section and a sole portion connecting the bottom medial section to the bottom lateral section. Further, the at least two draw straps connect the top section of the support frame to the bottom lateral section of the support frame on the lateral side of the ankle support. Such a one piece support frame extending under the foot from the medial side to the lateral side of the foot and the direct connection between the support frame and the draw straps on the lateral side ensures that the load belonging to the movement associated with the inversion is more directly transmitted from the draw straps to the support frame. Hence, the ankle support is further improved to more effectively prevent inversion of the foot.

Further, the sole portion of the support frame extending between the medial side of the foot and the lateral side of the foot provides comfort for a user wearing the ankle support. Under the sole of the foot there is no additional connection required for fixing the metal or plastic support frame in its longitudinal direction. Hence, the portion of the sole of the user contacting the ankle support only contacts the sole section of the one-piece support frame which is free of any transitions for fixing purposes, such as for example the pocket shown in figure 3 of EP-1.138.288. The sole section of the one-piece support frame also effectively fixes the top section and the connection section against movement in their longitudinal direction. Further, the design of the one-piece support frame also no longer requires a draw strap running over the foot instep which provides more comfort inside a shoe for a user and increases the freedom of movement.

The support frame may be made by multi-material injection molding, preferably sandwich molding. In this way it is possible to produce a one-piece support frame anatomically adapted to the contours of that section of the ankle, foot and leg along which it runs such that the support frame provides maximum freedom of movement in directions other than the inversion direction. Further, by using multi-material injection molding, preferably sandwich molding to produce the support frame it is possible to provide a semi-rigid frame which can be soft and comfortable for a user on the outside and provide the desired rigidity from inside to avoid inversion. Hence, the support frame comprises an outer layer and an inner core layer, wherein the inner core layer is made of a material less flexible than the material of the outer layer. The inner core layer can be made of fibre-reinforced plastic and the outer layer can be made of another plastic material, such as a more soft and/or a skin-friendly plastic. By varying the thickness of the core layer in the support frame it is possible to vary the rigidity in the support frame such that preventing inversion trauma versus user comfort of the ankle support can be further optimized. For example, the core layer may have a greater thickness in the connection section than in the top section and/or the core layer may have a greater thickness in the bottom medial section than the bottom lateral section.

In one aspect of the ankle support according to the present invention the circumferential edge of the support frame consists of the outer layer without the core layer. Such an circumferential edge of the support frame is more flexible such that it provides more comfort during wearing. Further, the circumferential edge of the support frame can be easily used for connecting, for example by stitching and/or bonding, other components of the ankle support such as for example gauze fabric sock-like portions of the ankle support to the support frame of the ankle support.

The connection section of the support frame comprises an upper portion, a lower portion and a central portion located between the upper portion and the lower portion, wherein the central portion has a smaller width than the lower portion and/or the upper portion such that when the ankle support is arranged on the foot, the connection section of the support frame has a configuration allowing maximal dorsiflexion and plantar flexion of the tibiotalar joint. The connection section runs past the medial malleolus, preferably the connection section is located on a dorsal side of the medial malleolus and/or the connection section is located adjacent to the medial malleolus. The connection section may also be wider in the central portion if desired, in which case the central portion can be provided with an opening which exposes the medial malleolus.

The bottom lateral section of the support frame is, when the ankle support is arranged on the foot, located under the lateral malleolus, i.e. seen in the cranial-caudal direction the bottom lateral section is located lower than the lateral malleolus. The bottom lateral section may also be a circumferential edge of the sole portion. The bottom lateral section can be somewhat curved with respect to the sole portion for connecting one of the ends of each draw strap to the support frame. The bottom lateral section may extend between 0,5-5 cm from the sole portion towards the lateral malleolus when the ankle support is arranged on the foot, more preferred between 0,5 and 3 cm. In the ankle support of the present invention the height of the support frame on the lateral side is relatively small compared with the height of the support frame on the medial side.

The at least two draw straps of the ankle support pass through a slot-like opening in the top section of the support frame and are then turned back, after which ultimately the turned-back section is fixed to the incoming part, for example by means of hook and loop fasteners. This makes fitting very easy and provides a great deal of flexibility in terms of adaptation to the dimensions of the foot and lower leg, which differ from person to person. In addition, the degree of support of the support frame can be individually adjusted. It is also possible that the draw straps are directly connected to the top section of the support frame, for example by means of press-studs, buckles or hook and loop fasteners. The draw straps consist of a material without elasticity or with very low elasticity, in order to prevent inversion of the foot.

Further, the two draw straps which allow dorsiflexion and plantar flexion of the foot comprise a dorsal draw strap and a ventral draw strap, wherein, when the ankle support is arranged on the foot, the dorsal draw strap extends substantially perpendicular to the subtalar joint axis. This position of the dorsal draw strap maximally prevents inversion of the foot during use of the ankle support. In addition, the two draw straps may cross each other on the lateral side of the ankle support. The crossing of the draw straps makes it possible to provide a spacious user-entry in the ankle support, because it is no longer required to use an ankle support having a sock which covers the lower leg and at least a portion of the foot. In this way it is possible to produce a more user-friendly ankle support which may be easily put on and taken off. Also, the crossing of the draw straps provides maximal dorsiflexion and plantar flexion of the tibiotalar joint, in particular when the two draw straps cross each other close to or in the tibiotalar joint axis. "Close to the tibiotalar joint axis" is intended to mean in this text that the crossing area of the straps lies in a distance from 0-2 cm from the tibiotalar joint axis.

The invention will now be explained in more detail on the basis of exemplary embodiments in the appended drawings, in which:
Figures 1a, 1b show a schematic view of a first embodiment of an ankle support, in particular the lateral side of the ankle support;
Figure 2 shows a schematic view of the ankle support shown in figure 1, in particular the medial side of the ankle support.
Figure 3 shows a schematic view of a second embodiment of an ankle support.

Like parts are indicated by the same numerals in the figures.

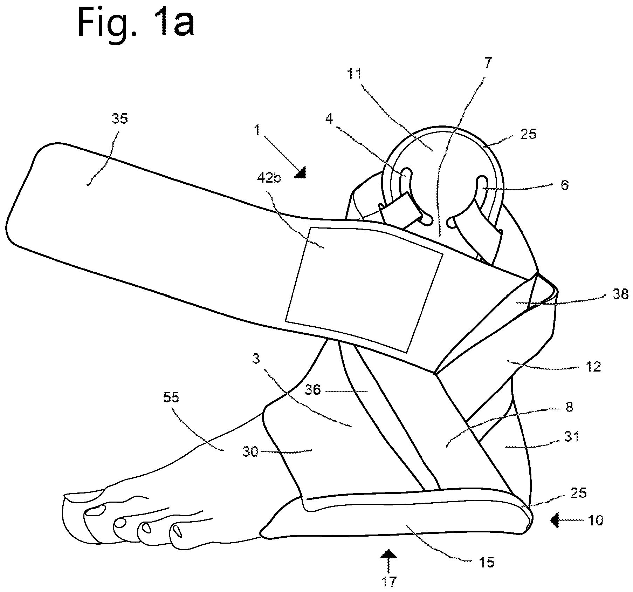

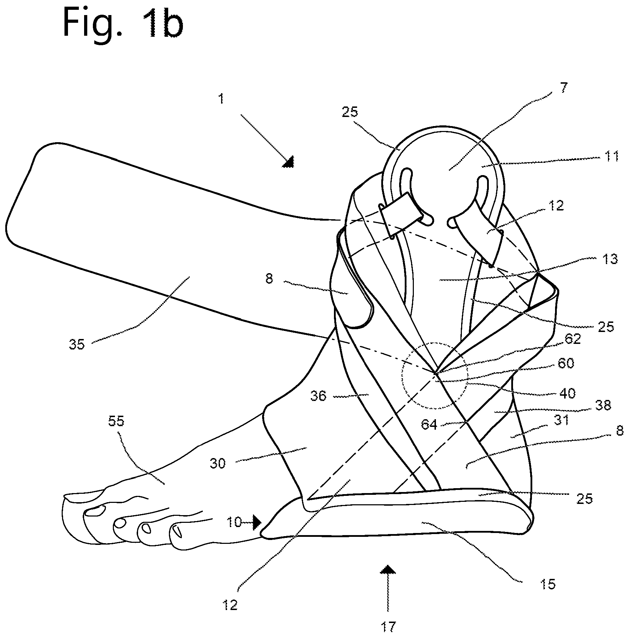

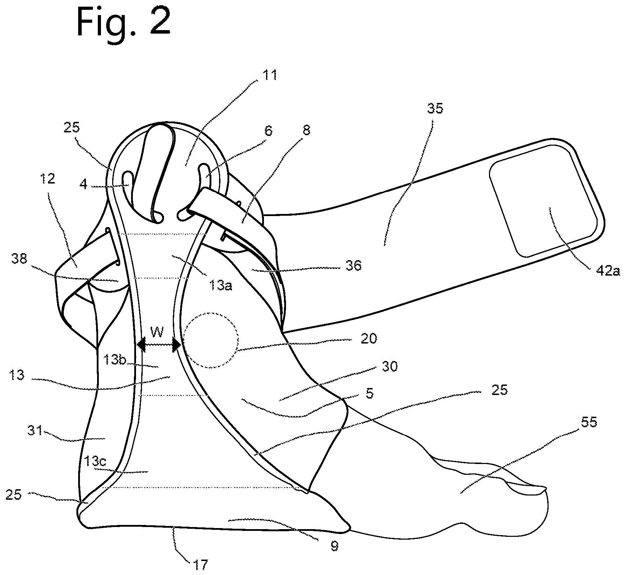

Figures 1a,b and 2 show a first embodiment of an ankle support 1 according to the present invention. Figures 1a,b show the same ankle support 1 from the same perspective, but in figure 1b the flexible compression strap 35, a draw strap 12 and the lateral malleolus 40 are (partly) indicated with broken lines. Further, the broken lines used in figure 2 illustrate the medial malleolus 20 and the various sections of the connection section. It may be understood that the broken lines are for illustrative purposes only.

The ankle support 1 has a lateral side 3 (shown in figures 1a,b) and a medial side 5 (shown in figure 2). The ankle support 1 comprises a one-piece support frame 7 with a bottom section 10, a top section 11 and a connection section 13.

The connection section 13 of the support frame 7 connects the top section 11 with the bottom section 10 on the medial side 5 of the ankle support 1, such that, when the ankle support is arranged on the lower leg, the ankle and the foot, the top section 11 of the support frame 7 is located above the medial malleolus 20. The medial malleolus 20 is located close to the middle (seen in the cranial-caudal direction) of the medial side of the support frame 7.

The bottom section 10 of the support frame 7 comprises a bottom medial section 9 connected to the connection section 13, a bottom lateral section 15 and a sole portion 17 connecting the bottom medial section 9 to the bottom lateral section 15. Below a portion of the foot 55, in particular between the posterior end of the foot 55 and the midfoot of the foot 55, a sole section 17 extends from the medial side 5 to the lateral side 3. The support frame 7 has a U-shaped bottom section 10, wherein the first leg of the U-shape is provided by the bottom medial section 9, the second leg is provided by the bottom lateral section 15 and the bridge connecting the first and second legs is provided by the sole portion 17. The support frame 7 does not cover the posterior ankle.

As shown in figure 1b, the bottom lateral section 15 of the support frame 7 is located under the lateral malleolus 40 (broken lines in figure 1b). The bottom lateral section 15 extends approximately 1 cm from the sole portion 17 towards the lateral malleolus 40.

The ankle support 1 further has at least two draw straps 8, 12, i.e. a dorsal draw strap 12 and a ventral draw strap 8, which, when the ankle support is arranged on the foot which, each run along different sides of the lower leg.

Each draw strap 8, 12 of the ankle support 1 passes through a slot-like opening 4, 6 in the top section 11 of the support frame 7 and is then turned back, after which ultimately the turned-back section is fixed to the incoming part, for example by means of hook and loop fasteners (not shown). This makes fitting of the draw straps to customize the ankle support to the personal dimensions (and preferences) of the user very easy.

As can be seen in figure 1b the two draw straps 8, 12 cross each other on the lateral side of the ankle support 1, preferably the at least two draw straps 8, 12 cross each other close to the tibiotalar joint axis 60 to provide maximal dorsiflexion and plantar flexion of the tibiotalar joint.

When the ankle support 1 is arranged on the foot, the dorsal draw strap 12 extends substantially perpendicular to the subtalar joint axis. This position of the dorsal draw strap 12 maximally prevents inversion of the foot 55 during use of the ankle support 1. The ventral draw strap 8 ensures a symmetrical load to the support frame 7, such that in use a rotation component to the lower leg can be avoided.

The two draw straps 8, 12 further connect the top section 11 of the support frame 7 to the bottom lateral section 15 of the support frame 7 on the lateral side of the ankle support. As shown in figure 1b, the ends of the draw straps 8, 12 are connected on the lateral side 3 to the bottom lateral section 15 of the support frame 7, for example by stitching and/or bonding.

The direct connection of the one-piece support frame 7 and the draw straps 8, 12 without any other components of the ankle support there between increase the main function of the ankle support 1: preventing inversion of the foot, i.e. the direct connection increases the response time of the ankle support 1 to the slightest inversion of the foot, immediately preventing further movement in the inversion direction. In addition, the one-piece support frame 7 maximises usercomfort, because the load bearing surfaces of the foot only make contact with the outer material of the sole portion of the one-piece support frame 7. This sole portion 17 also longitudinally (cranial-caudal direction) fixes the elongate connection section 13 and top section 11 of the support frame 7. The elongate connection section 13 of the support frame 7 should be sufficiently deformable perpendicular to the longitudinal direction to anatomically adapt itself to the contours of that section of the foot and leg along which it runs, but also does not bend in its longitudinal direction when a load is imposed on the support frame 7 by the draw straps 8, 12. The support frame 7 is fixed in its longitudinal direction by means of the bottom section 10 of the support frame 7.

Figure 2 shows that the connection section 13 runs past the medial malleolus 20, and that the connection section 13 is located on a dorsal side of the medial malleolus 20, adjacent to the medial malleolus 20. As shown with broken lines in figure 2, the connection section 13 comprises an upper portion 13a, a lower portion 13c and a central portion 13b located between the upper portion 13a and the lower portion 13c, wherein the central portion 13b has a smaller width (W) than the lower portion 13c and/or the upper portion 13a such that the connection section 13 of the support frame 7 has a configuration allowing maximal dorsiflexion and plantar flexion of the foot 55, in particular maximal dorsiflexion and plantar flexion of the tibiotalar joint (not shown). The central section 13b has a concave shape on the side facing the medial malleolus 20.

The ankle support 1 comprises two separate sock-like portions 30, 31 which are connected to each other by the support frame 7, preferably by bonding and/or stitching the circumferential edges of the two separate sock-like portions 30, 31 to the circumferential edge 25 of the support frame 7. It is also possible to use a textile band bonded to the circumferential edge of the each separate sock-like portion 30, 31 to be connected to the support frame 7, wherein the band enclosing to the circumferential edge of the sock-like portion 30, 31 is stitched to the circumferential edge 25 of the support frame 7. The two separate sock-like portions 30, 31 only overlap each other near the bottom lateral section 15 of the support frame 7, wherein the overlapping sock-like portions 30, 31 are separately connected to the circumferential edge 25 of the support frame 7. Each sock-like portion 30, 31 is further connected to a fabric strap 36, 38. These fabric strips 36, 38 are located mainly under the draw straps 8, 12 and are further connected to the support frame 7. Towards the bottom lateral section 15 the draw straps 8, 12 are connected (stitched/bonded) to the fabric strips 36, 38, wherein approximately above the crossing area of the draw straps 8, 12 (indicated by reference sign 62), the draw straps are no longer connected to the fabric strips 36, 38 and the draw straps loosely lie on these fabric strips 36, 38. These fabric strips 36, 38 lying below the draw straps increase the comfort of the ankle support 1 if the draw straps 8, 12 are tensioned for use of the ankle support 1. It is of course possible to provide an ankle support without these fabric strips 36, 38.

The two separate sock-like portions 30, 31 comprise a ventral portion 30 and a dorsal portion 31, wherein the ventral portion 30 covering the foot instep is located below the draw straps 8, 12. Hence, the foot instep is free of draw straps 8, 12 which increases the comfort of the ankle support 1 in a shoe and increases the freedom of movement.

An entry opening (best shown in figure 1b) for a foot of a user, is defined by the support frame 7 and by the non-elastic draw straps 8, 12 loosely located on the fabric strips 36, 38, wherein the lowest point (indicated by reference sign 64) of the entry opening is defined by the two draw straps 8, 12 crossing each other on the lateral side of the ankle support 1. Between the points 62 and 64 as indicated in figure 1b the strap 8 and the fabric strip 36 are located loosely on the draw strap 12, wherein the fabric strips 36, 38 and the draws straps 8, 12 are connected (stitched) to each other in point 64.

The ankle support 1 further comprises a third elastic strap 35 attached to the connection section 13 of the support frame 7, wherein the third strap is more elastic than the draw straps 8, 12. The third strap 35 comprises hook and loop fasteners 42a, 42b which can be connected to each other by a user after wrapping the third strap 35 around the lower leg. The third strap 35 is elastic and can be tensioned around the lower leg such that the third strap 35 is able to provide compression to the lower leg. In addition, the third strap 35 encloses the turned-back sections of the draw strips 8, 12 which are detachably fixed to the incoming parts of the draw strips 8, 12 such that in use of the ankle support 1 the third strap 35 prevents loosening of the detachable connections of the draw strips 8, 12.

Further, the third elastic strap 35 can be connected (stitched) to the draw strip 12 on or close to (on the dorsal side of) point 62 shown in figure 2. This connection provides the advantage that wrapping and tensioning the third strap 35 around the leg also ensures that the dorsal draw strip 12 is correctly positioned and remains correctly positioned with respect to the subtalar joint axis (the dorsal draw strap 12 should extend substantially perpendicular to the subtalar joint axis) and/or the tibiotalar joint axis. The elastic strap 35 is preferably on its inner side (shown in figure 1b) provided with silicon strips (not shown) extending in the longitudinal direction of the elastic strap 35. These silicon strips provide a surface with high friction such that after wrapping and tensioning the elastic strap 35 around the lower leg, the elastic strap 35 also fixates the positions and orientation of the draw straps 8, 12 during use of the ankle support 1.

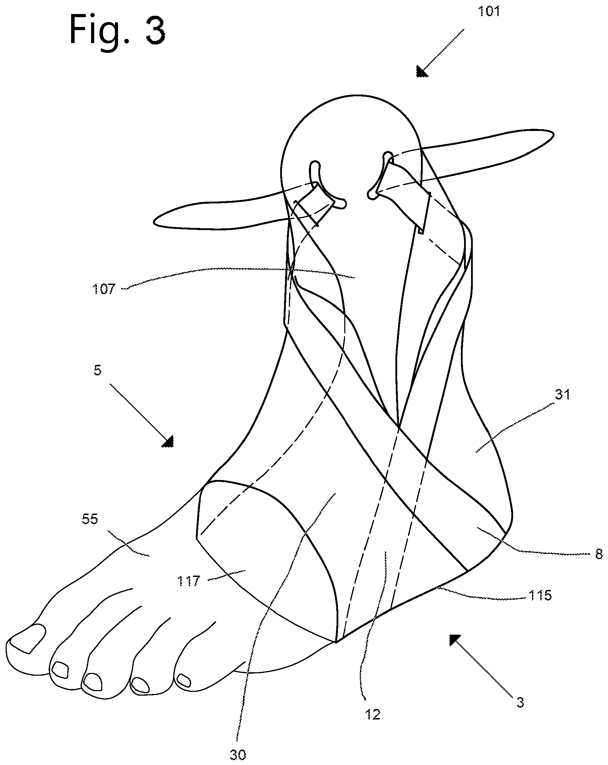

The embodiment of the ankle support 101 shown in figure 3 minimally differs from the ankle support 1 shown in figures 1a,b and 2. The main difference is the support frame 107 which differs from the support frame 7 of the ankle support 1 shown in figures 1a, band 2, in that the bottom lateral section 115 of the support frame 107 is a circumferential edge of the sole portion 117, i.e. the bottom lateral section 115 extends minimally, i.e. less than 0,5 cm from the sole portion 117 towards the lateral malleolus (not shown in figure 3). Hence, the support frame 107 has a L-shaped bottom section, wherein the first leg of the L-shape is provided by the bottom medial section (not shown) and the second leg is provided by the sole portion 117 and the bottom lateral section 115 (mainly by the sole portion 117).

The support frame 7, 107 is made by multi-material injection molding, preferably sandwich molding, to produce a relatively thin one-piece support frame 7, 107 anatomically adapted to the contours of that section of the ankle, foot and leg along which it runs. Such a support frame 7, 107 should be thin enough that the ankle support 1, 101 can be worn in a shoe.

The support frame 7, 107 comprises an outer layer and an inner core layer, wherein the inner core layer is made of a material less flexible than the material of the outer layer. The inner core layer can be made of fibre-reinforced plastic and the outer layer can be made of another plastic material, such as a more soft and/or a skin-friendly plastic. By varying the thickness of the core layer in the support frame 7, 107 it is possible to vary the flexibility in the support frame 7, 107 such that main function preventing inversion trauma can be maximized and user comfort of the ankle support 1, 101 can be maximized. For example, the core layer may have a greater thickness in the connection section 13 than in the top section 11 and/or the core layer may have a greater thickness in the bottom medial section 9 than the bottom lateral section 15, 115.

The circumferential edge 25 of the support frame 7, 107 consists of the outer layer without the core layer. Such an circumferential edge of the support frame is more flexible such that it provides more comfort during wearing. Further, the circumferential edge 25 of the support frame 7, 107 can be used for connecting, for example by stitching and/or bonding, other components of the ankle support such as for example gauze fabric sock-like portions 30, 31 of the ankle support 1, 101 to the support frame 7, 107 of the ankle support 1, 101.

## Claims

1. Ankle support (1; 101) having a lateral side (3) and a medial side (5), at least comprising:
- a one-piece support frame (7; 107) with a bottom section, a top section (11) and a connection section (13), the connection section connects the top section with the bottom section on the medial side of the ankle support, such that, when the ankle support is arranged on the lower leg, the ankle and the foot, the top section of the support frame is adapted to be located above the medial malleolus (20),
- at least two draw straps (8, 12) which, when the ankle support is arranged on the foot, each run along different sides of the lower leg, wherein
the bottom section of the support frame comprises a bottom medial section (9) connected to the connection section (13), a bottom lateral section (15; 115) and a sole portion (17; 117) connecting the bottom medial section to the bottom lateral section, wherein the at least two draw straps connect the top section of the support frame to the bottom lateral section of the support frame on the lateral side of the ankle support, **characterized in that**
the at least two draw straps (8, 12) cross each other on the lateral side of the ankle support (1).

2. Ankle support (1; 101) according to claim 1, wherein the support frame is made by multi-material injection molding, preferably sandwich molding.

3. Ankle support (1; 101) according to claim 1 or 2, wherein the support frame comprises an outer layer and an inner core layer, wherein the inner core layer is made of a material less flexible than the material of the outer layer, preferably the inner core layer is made of fibre-reinforced plastic and the outer layer is made of another plastic material, preferably a more soft and/or a skin-friendly plastic.

4. Ankle support (1; 101) according to claim 3, wherein the thickness of the core layer in the support frame varies, preferably the core layer has a greater thickness in the connection section than in the top section and/or the core layer has a greater thickness in the bottom medial section than the bottom lateral section.

5. Ankle support (1; 101) according to claim 3 or 4, wherein the circumferential edge (25) of the support frame consists of the outer layer without the core layer.

6. Ankle support (1; 101) according to one of the preceding claims, wherein the bottom lateral section of the support frame is a circumferential edge of the sole portion, and/or when the ankle support is arranged on the foot, the bottom lateral section of the support frame is adapted to be located under the lateral malleolus (40), and/or the bottom lateral section is adapted to extend between 0,5-5 cm from the sole portion towards the lateral malleolus when the ankle support is arranged on the foot, preferably between 0,5 and 3 cm.

7. Ankle support (1; 101) according to one of the preceding claims, wherein the connection section comprises an upper portion, a lower portion and a central portion located between the upper portion and the lower portion, wherein the central portion has a smaller width (W) than the lower portion and/or the upper portion such that when the ankle support is arranged on the foot, the connection section of the support frame has a configuration allowing dorsiflexion and plantar flexion of the foot.

8. Ankle support (1; 101) according to one of the preceding claims, wherein, when the ankle support is arranged on the foot, the connection section is adapted to run past the medial malleolus (20), preferably the connection section is adapted to be located on a dorsal side of the medial malleolus (20), preferably the connection section is adapted to be located adjacent to the medial malleolus (20).

9. Ankle support (1; 101) according to one of the preceding claims, wherein the at least two draw straps (8, 12) are adapted to cross each other close to or in the tibiotalar joint axis.

10. Ankle support (1; 101) according to one of the preceding claims, wherein the at least two draw straps (8, 12) which allow dorsiflexion and plantar flexion of the foot comprise a dorsal draw strap (12) and a ventral draw strap (8), wherein, when the ankle support is arranged on the foot, the dorsal draw strap is adapted to extend substantially perpendicular to the subtalar joint axis, and/or wherein, when the ankle support is arranged on the foot, the ventral draw strap is configured to provide a symmetrical load to the support frame.

11. Ankle support (1; 101) according to one of the preceding claims, wherein the ankle support comprises two separate sock-like portions (30, 31) which are connected to each other by the support frame, preferably by bonding and/or stitching.

12. Ankle support (1; 101) according to one of the preceding claims, wherein the ankle support comprises a third strap (35) attached to the connection section of the support frame, wherein the third strap is more elastic than the draw straps, wherein the third strap is adapted to mainly run around the lower leg.

13. Ankle support (1; 101) according to claim 11, wherein the two separate sock-like portions (30, 31) comprise a ventral portion and a dorsal portion, wherein the ventral portion covering the foot instep is located below the draw straps and/or the ventral portion covering the foot instep is located below the third strap.

14. Ankle support (1; 101) according to one of the preceding claims, wherein the ankle support comprises an entry opening for a foot of a user, wherein the entry opening of the foot is defined by the draw straps and the support frame, preferably the lowest point of the entry opening is defined by the two draw straps crossing each other on the lateral side of the ankle support (1).

15. Method for manufacturing the ankle support (1; 101) according to any of the preceding claims, wherein a support frame is made by multi-material injection molding, preferably sandwich molding.

## Patentansprüche

1. Sprunggelenkstütze (1; 101) mit einer lateralen Seite (3) und einer medialen Seite (5), mindestens umfassend:
- einen einteiligen Stützrahmen (7; 107) mit einem unterem Abschnitt, einem oberen Abschnitt (11) und einem Verbindungsabschnitt (13), wobei der Verbindungsabschnitt den oberen Abschnitt mit dem unteren Abschnitt auf der medialen Seite der Sprunggelenkstütze verbindet, so dass, wenn die Sprunggelenkstütze an dem Unterschenkel, dem Sprunggelenk und dem Fuß angeordnet ist, der obere Abschnitt des Stützrahmens dazu angepasst ist, sich über dem medialen Malleolus (20) zu befinden,
- mindestens zwei Zugbänder (8, 12), die, wenn die Sprunggelenkstütze an dem Fuß angeordnet ist, jeweils an verschiedenen Seiten des Unterschenkels entlang verlaufen, wobei
der untere Abschnitt des Stützrahmens einen unteren medialen Abschnitt (9) umfasst, der mit dem Verbindungsabschnitt (13) verbunden ist, einen unteren lateralen Abschnitt (15; 115) und einen Sohlenabschnitt (17; 117), der den unteren medialen Abschnitt mit dem unteren lateralen Abschnitt verbindet, wobei die mindestens zwei Zugbänder den oberen Abschnitt des Stützrahmens mit dem unteren lateralen Abschnitt des Stützrahmens auf der lateralen Seite der Sprunggelenkstütze verbinden, **dadurch gekennzeichnet, dass**
die mindestens zwei Zugbänder (8, 12) einander auf der lateralen Seite der Sprunggelenkstütze (1) überkreuzen.

2. Sprunggelenkstütze (1; 101) nach Anspruch 1, wobei der Stützrahmen durch Multi-Material-Spritzguss, vorzugsweise Sandwich-Spritzguss, hergestellt ist.

3. Sprunggelenkstütze (1; 101) nach Anspruch 1 oder 2, wobei der Stützrahmen eine äußere Schicht und eine innere Kernschicht umfasst, wobei die innere Kernschicht aus einem Material hergestellt ist, das weniger flexibel ist als das Material der äußeren Schicht, wobei die innere Kernschicht vorzugsweise aus faserverstärktem Kunststoff hergestellt ist und die äußere Schicht aus einem anderen Kunststoffmaterial, vorzugsweise einem weicheren und/oder einem hautfreundlichen Kunststoff hergestellt ist.

4. Sprunggelenkstütze (1; 101) nach Anspruch 3, wobei die Dicke der Kernschicht in dem Stützrahmen variiert, wobei die Kernschicht vorzugsweise eine größere Dicke in dem Verbindungsabschnitt als in dem oberen Abschnitt hat und/oder die Kernschicht eine größere Dicke in dem unteren medialen Abschnitt als in dem unteren lateralen Abschnitt hat.

5. Sprunggelenkstütze (1; 101) nach Anspruch 3 oder 4, wobei der Umfangsrand (25) des Stützrahmens aus der äußeren Schicht ohne die Kernschicht besteht.

6. Sprunggelenkstütze (1; 101) nach einem der vorhergehenden Ansprüche, wobei der untere laterale Abschnitt des Stützrahmens ein Umfangsrand des Sohlenabschnitts ist und/oder wenn die Sprunggelenkstütze an dem Fuß angeordnet ist, der untere laterale Abschnitt der Sprunggelenkstütze dazu angepasst ist, sich unter dem lateralen Malleolus (40) zu befinden, und/oder der untere laterale Abschnitt dazu angepasst ist, sich zwischen 0,5-5 cm von dem Sohlenabschnitt in Richtung des lateralen Malleolus zu erstrecken, wenn die Sprunggelenkstütze an dem Fuß angeordnet ist, vorzugsweise zwischen 0,5 und 3 cm.

7. Sprunggelenkstütze (1; 101) nach einem der vorhergehenden Ansprüche, wobei der Verbindungsabschnitt einen oberen Teil, einen unteren Teil und einen mittleren Teil, der zwischen dem oberen Teil und dem unteren Teil befindlich ist, umfasst, wobei der mittlere Teil eine kleinere Breite (W) als der untere Teil und/oder der obere Teil hat, so dass, wenn die Sprunggelenkstütze an dem Fuß angeordnet ist, der Verbindungsabschnitt des Stützrahmens eine Auslegung hat, die Dorsalflexion und Plantarflexion des Fußes ermöglicht.

8. Sprunggelenkstütze (1; 101) nach einem der vorhergehenden Ansprüche, wobei, wenn die Sprunggelenkstütze an dem Fuß angeordnet ist, der Verbindungsabschnitt dazu angepasst ist, an dem medialen Malleolus (20) vorbeizulaufen, wobei der Verbindungsabschnitt vorzugsweise dazu angepasst ist, sich auf einer dorsalen Seite des medialen Malleolus (20) zu befinden, wobei der Verbindungsabschnitt vorzugsweise dazu angepasst ist, sich neben dem medialen Malleolus (20) zu befinden.

9. Sprunggelenkstütze (1; 101) nach einem der vorhergehenden Ansprüche, wobei die mindestens zwei Zugbänder (8, 12) dazu angepasst sind, einander nahe der oder in der Tibiotalargelenkachse zu überkreuzen.

10. Sprunggelenkstütze (1; 101) nach einem der vorhergehenden Ansprüche, wobei die mindestens zwei Zugbänder (8, 12), die Dorsalflexion und Plantarflexion des Fußes ermöglichen, ein dorsales Zugband (12) und ein ventrales Zugband (8) umfassen, wobei, wenn die Sprunggelenkstütze an dem Fuß angeordnet ist, das dorsale Zugband dazu angepasst ist, sich im Wesentlichen senkrecht zu der Subtalargelenkachse zu erstrecken, und/oder wobei, wenn die Sprunggelenkstütze an dem Fuß angeordnet ist, das ventrale Zugband dazu ausgelegt ist, eine symmetrische Belastung auf den Stützrahmen bereitzustellen.

11. Sprunggelenkstütze (1; 101) nach einem der vorhergehenden Ansprüche, wobei die Sprunggelenkstütze zwei separate sockenähnliche Teile (30, 31) umfasst, die durch den Stützrahmen miteinander verbunden sind, vorzugsweise durch Verkleben und/oder Nähen.

12. Sprunggelenkstütze (1; 101) nach einem der vorhergehenden Ansprüche, wobei die Sprunggelenkstütze ein drittes Band (35) umfasst, das an dem Verbindungsabschnitt des Stützrahmens angebracht ist, wobei das dritte Band elastischer ist als die Zugbänder, wobei das dritte Band dazu angepasst ist, hauptsächlich rund um den Unterschenkel zu verlaufen.

13. Sprunggelenkstütze (1; 101) nach Anspruch 11, wobei die zwei separaten sockenähnlichen Teile (30, 31) einen ventralen Teil und einen dorsalen Teil umfassen, wobei der ventrale Teil, der den Fußrist abdeckt, sich unter den Zugbändern befindet, und/oder der ventrale Teil, der den Fußrist abdeckt, sich über dem dritten Band befindet.

14. Sprunggelenkstütze (1; 101) nach einem der vorhergehenden Ansprüche, wobei die Sprunggelenkstütze eine Einfahröffnung für einen Fuß eines Anwenders umfasst, wobei die Einfahröffnung des Fußes durch die Zugbänder und den Stützrahmen definiert wird, wobei vorzugsweise der tiefste Punkt der Einfahröffnung durch die zwei Zugbänder definiert wird, die einander auf der lateralen Seite der Sprunggelenkstütze (1) kreuzen.

15. Verfahren zur Herstellung der Sprunggelenkstütze (1; 101) nach einem der vorhergehenden Ansprüche, wobei ein Stützrahmen durch Multi-Material-Spritzguss, vorzugsweise Sandwich-Spritzguss, hergestellt wird.

## Revendications

1. Support de cheville (1 ; 101) ayant un côté latéral (3) et un côté médial (5), comprenant au moins :
- un cadre de support (7 ; 107) monobloc avec une section inférieure, une section supérieure (11) et une section de liaison (13), la section de liaison relie la section supérieure à la section inférieure sur le côté médial du support de cheville, de sorte que, lorsque le support de cheville est agencé sur la jambe inférieure, la cheville et le pied, la section supérieure du cadre de support soit adaptée pour être située au-dessus de la malléole médiale (20),
- au moins deux sangles de traction (8, 12) qui, lorsque le support de cheville est agencé sur le pied, s'étendent chacune le long de différents côtés de la jambe inférieure, dans lequel la section inférieure du cadre de support comprend une section médiale inférieure (9) reliée à la section de liaison (13), une section latérale inférieure (15 ; 115) et une partie plante (17 ; 117) reliant la section médiale inférieure à la section latérale inférieure, où les au moins deux sangles de traction relient la section supérieure du cadre de support à la section latérale inférieure du cadre de support sur le côté latéral du support de cheville, **caractérisé en ce que** les au moins deux sangles de traction (8, 12) se croisent sur le côté latéral du support de cheville (1).

2. Support de cheville (1 ; 101) selon la revendication 1, dans lequel le cadre de support est réalisé par moulage par injection multi-matériaux, de préférence par moulage sandwich.

3. Support de cheville (1 ; 101) selon la revendication 1 ou 2, dans lequel le cadre de support comprend une couche externe et une couche centrale interne, où la couche centrale interne est réalisée en un matériau moins flexible que le matériau de la couche externe, de préférence la couche centrale interne est réalisée en une matière plastique renforcée par des fibres et la couche externe est réalisée en une autre matière plastique, de préférence en une matière plastique plus souple et/ou douce pour la peau.

4. Support de cheville (1 ; 101) selon la revendication 3, dans lequel l'épaisseur de la couche centrale dans le cadre de support varie, de préférence la couche centrale a une épaisseur plus grande dans la section de liaison que dans la section supérieure et/ou la couche centrale a une épaisseur plus grande dans la section médiale inférieure que dans la section latérale inférieure.

5. Support de cheville (1 ; 101) selon la revendication 3 ou 4, dans lequel le bord circonférentiel (25) du cadre de support est constitué de la couche externe sans la couche centrale.

6. Support de cheville (1 ; 101) selon l'une des revendications précédentes, dans lequel la section latérale inférieure du cadre de support est un bord circonférentiel de la partie plante, et/ou lorsque le support de cheville est agencé sur le pied, la section latérale inférieure du cadre de support est adaptée pour être située sous la malléole latérale (40), et/ou la section latérale inférieure est adaptée pour s'étendre entre 0,5-5 cm de la partie plante vers la malléole latérale lorsque le support de cheville est agencé sur le pied, de préférence entre 0,5 et 3 cm.

7. Support de cheville (1 ; 101) selon l'une des revendications précédentes, dans lequel la section de liaison comprend une partie supérieure, une partie inférieure et une partie centrale située entre la partie supérieure et la partie inférieure, où la partie centrale a une largeur (W) inférieure à celle de la partie inférieure et/ou de la partie supérieure de sorte que, lorsque le support de cheville est agencé sur le pied, la section de liaison du cadre de support ait une configuration permettant la dorsiflexion et la flexion plantaire du pied.

8. Support de cheville (1 ; 101) selon l'une des revendications précédentes, dans lequel, lorsque le support de cheville est agencé sur le pied, la section de liaison est adaptée pour passer devant la malléole médiale (20), de préférence la section de liaison est adaptée pour être située sur un côté dorsal de la malléole médiale (20), de préférence la section de liaison est adaptée pour être située de manière adjacente à la malléole médiale (20).

9. Support de cheville (1 ; 101) selon l'une des revendications précédentes, dans lequel les au moins deux sangles de traction (8, 12) sont adaptées pour se croiser à proximité de ou dans l'axe de l'articulation tibio-tarsienne.

10. Support de cheville (1 ; 101) selon l'une des revendications précédentes, dans lequel les au moins deux sangles de traction (8, 12) qui permettent la dorsiflexion et la flexion plantaire du pied comprennent une sangle de traction dorsale (12) et une sangle de traction ventrale (8), où, lorsque le support de cheville est agencé sur le pied, la sangle de traction dorsale est adaptée pour s'étendre sensiblement perpendiculairement à l'axe de l'articulation sous-astragalienne, et/ou où, lorsque le support de cheville est agencé sur le pied, la sangle de traction ventrale est configurée pour fournir une charge symétrique au cadre de support.

11. Support de cheville (1 ; 101) selon l'une des revendications précédentes, dans lequel le support de cheville comprend deux parties séparées en forme de chaussette (30, 31) qui sont reliées l'une à l'autre par le cadre de support, de préférence par collage et/ou couture.

12. Support de cheville (1 ; 101) selon l'une des revendications précédentes, dans lequel le support de cheville comprend une troisième sangle (35) fixée à la section de liaison du cadre de support, où la troisième sangle est plus élastique que les sangles de traction, où la troisième sangle est adaptée pour entourer principalement la jambe inférieure.

13. Support de cheville (1 ; 101) selon la revendication 11, dans lequel les deux parties séparées en forme de chaussette (30, 31) comprennent une partie ventrale et une partie dorsale, où la partie ventrale recouvrant le cou-de-pied est située sous les sangles de traction et/ou la partie ventrale recouvrant le cou-de-pied est située sous la troisième sangle.

14. Support de cheville (1 ; 101) selon l'une des revendications précédentes, dans lequel le support de cheville comprend une ouverture d'entrée pour un pied d'un utilisateur, où l'ouverture d'entrée du pied est définie par les sangles de traction et le cadre de support, de préférence le point le plus bas de l'ouverture d'entrée est défini par les deux sangles de traction se croisant sur le côté latéral du support de cheville (1).

15. Procédé de fabrication du support de cheville (1 ; 101) selon l'une des revendications précédentes, dans lequel un cadre de support est réalisé par moulage par injection multi-matériaux, de préférence par moulage sandwich.
